(19) Europäisches Patentamt
European Patent Office
Office européen des brevets

(11) EP 3 127 556 A1

(12) **EUROPEAN PATENT APPLICATION**

(43) Date of publication:
08.02.2017 Bulletin 2017/06

(21) Application number: 15180256.8

(22) Date of filing: 07.08.2015

(51) Int Cl.:
A61K 47/10 (2017.01)　　　A61K 47/14 (2017.01)
A61K 9/14 (2006.01)　　　A61K 9/48 (2006.01)
A61K 31/192 (2006.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA

(71) Applicant: InFirst Healthcare Limited
London
WC2R 1BH (GB)

(72) Inventors:
• BANNISTER, Robin Mark
London, EC2Y 8AD (GB)
• BREW, John
London, EC2Y 8AD (GB)
• REILEY, Richard Robert
London, EC2Y 8AD (GB)
• CAPAROS-WANDERLEY, Wilson
London, EC2Y 8AD (GB)

(74) Representative: Schlich, George et al
Schlich
9 St Catherine's Road
Littlehampton, West Sussex BN17 5HS (GB)

(54) **SOLID SOLUTION COMPOSITIONS FOR NSAID**

(57)　　A pharmaceutical composition for treatment of inflammation or pain or chronic inflammation is liquid at normal human body temperature, namely 37°C, solid at room temperature, namely 20°C, and comprises:
a) 25% to 31 % by weight of a non-steroidal anti-inflammatory drug (NSAID);
b) 34% to 40% by weight of a lipid which is solid at room temperature, namely 20°C;
c) 22% to 28% by weight of a lipid which is a liquid at room temperature, namely 20°C; and
d) 7% to 13% by weight of a stabilizing agent comprising liquid glycol polymer.

Fig. 1A

Fig. 1B

## Description

Field of the Invention

The present invention relates to compositions comprising NSAIDs for the treatment of inflammation, pain and/or chronic inflammation. The compositions are typically solid solutions.

Background

Classic solid solution compositions are crystalline solids comprising a matrix of a solvent material (which may be a solid at normal temperatures) and solutes where the molecules in the solid solution are arranged in a random fashion and not in an ordered alignment.

Solid solution pharmaceutical compositions act as a delivery system that enables a therapeutic compound to be more effectively delivered or targeted to a cell type, tissue, organ, or region of the body that more effectively inhibits a pro-inflammatory response.

Solid solution compositions formulated using a lipid-soluble drug formulation only require a lipid component to formulate a therapeutic compound into a solution composition. Typically a hydrophilic solvent is absent or effectively absent. Without wishing to be limited by a theory, lipid-soluble drugs can typically be dissolved in a lipid under heat. Upon cooling it is believed that the lipid component and the drug form lipid-drug matrices organised in a manner such that the lipids encase the drug. Since only hydrophobic interactions are present, there is no organized alignment of these lipid-drug matrices resulting in a solid solution composition (i.e. there is no crystallization into a classic solid form).

Object of the Invention

One object of the invention is to provide an improved solid solution composition comprising an NSAID.

Another objection of the invention is to provide an improved formulation for use in therapy for treating inflammation, pain or chronic inflammation.

Summary

The present invention discloses solid solution pharmaceutical compositions, formulated in a manner that produces a lipid-adjuvant delivery system that enables NSAIDs to be delivered in a manner that more effectively inhibits a pro-inflammatory response. The end result is an improved treatment for inflammation, pain and/or chronic inflammation.

According to the invention there is provided a pharmaceutical composition comprising:

   i) 25% to 31% by weight of a non-steroidal anti-inflammatory drug (NSAID);
   ii) 34% to 40% by weight of a lipid which is solid at room temperature, namely 20°C;
   iii) 22% to 28% by weight of a lipid which is a liquid at room temperature, namely 20°C; and
   iv) 7% to 13% by weight of a stabilizing agent comprising liquid glycol polymer.

The compositions are preferably solid at typical room temperature, which for the purpose of this invention is defined as 20°C, but melt at normal body temperature, i.e. once ingested, typically 37°C. Thus the compositions may have a melting point of 28-36°C, typically 30-34°C.

In use, the resulting melted composition readily forms micelles which may be absorbed by the intestine, assembled into chylomicrons, and ultimately may be absorbed by macrophages or taken up by dendritic cells. This leads to improved absorption and improved effects of the NSAID.

NSAIDs are a large group of therapeutic compounds with analgesic, anti-inflammatory, and anti-pyretic properties. NSAIDs reduce inflammation by blocking cyclooxygenase. NSAIDs can be a non-selective cyclo-oxygenase (COX) inhibitor, a selective cyclooxygenase 1 (COX 1) inhibitor, a selective cyclooxygenase 2 (COX 2) inhibitor. NSAIDs include salicylate derived NSAIDs, p-amino phenol derived NSAIDs, propionic acid derived NSAIDs, acetic acid derived NSAIDs, enolic acid (Oxicam) derived NSAIDs and fenamic acid derived NSAIDs. NSAIDs include, without limitation, Aceclofenac, Acemetacin, Actarit, Alcofenac, Alminoprofen, Amfenac, Aloxiprin, Aminophenazone, Antraphenine, Aspirin, Azapropazone, Benorilate, Benoxaprofen, Benzydamine, Butibufen, Celecoxib, Chlorthenoxacin, Choline Salicylate, Clometacin, Dexketoprofen, Diclofenac, Diflunisal, Emorfazone, Epirizole; Etodolac, Etoricoxib, Feclobuzone, Felbinac, Fenbufen, Fenclofenac, Flurbiprofen, Glafenine, Hydroxylethyl salicylate, Ibuprofen, Indometacin, Indoprofen, Ketoprofen, Ketorolac, Lactyl phenetidin, Loxoprofen, Lumiracoxib, Mefenamic acid, Meloxicam, Metamizole, Metiazinic acid, Mofebutazone, Mofezolac, Nabumetone, Naproxen, Nifenazone, Niflumic acid, Oxametacin, Phenacetin, Pipebuzone, Pranoprofen, Propyphenazone, Proquazone, Protizinic acid, Rofecoxib, Salicylamide, Salsalate, Sulindac, Suprofen, Tiaramide, Tinoridine, Tolfenamic acid, Valdecoxib, and Zomepirac. Preferably, the active used in compositions of the invention is a derivative of 2-aryl propionate, e.g. ketoprofen, ibuprofen, flurbiprofen and naproxen. However, the most preferred NSAID is ibuprofen. Very suitably, the amount of NSAID is from about 26% to about 30% by weight of the composition, preferably from about 27% to about 29% by weight of the composition, and about 28% of the composition in a specific example described in more detail below.

Compositions of the invention can be made without the need for a hydrophilic solvent. In preferred embodiments, the compositions comprise 0.5% by weight or less of a hydrophilic solvent, more preferably 0.2% by weight or less of a hydrophilic solvent. More preferably the compositions are substantially free of hydrophilic solvent. In a specific example set out below, the composition is free of such a solvent.

A lipid may be broadly defined as a hydrophobic or amphiphilic small molecule. The amphiphilic nature of some lipids allows them to form structures such as vesicles, liposomes, or membranes in an aqueous environment. Non-limiting examples, of lipids include fatty acids, glycerolipids, phospholipids, sphingolipids, sterol lipids, prenol lipids, saccharolipids, and polyketides.

The chosen lipids must be "pharmaceutically acceptable" by which it is mean any molecular entity or composition that does not produce an adverse, allergic or other untoward or unwanted reaction when administered to an individual.

Very suitably, the amount of the lipid component that is solid at room temperature is from about 35% to about 39% by weight of the composition, more preferably from about 36% to about 38% by weight of the composition, and about 37% by weight in a specific example described in more detail below.

Suitable lipids which are solid at room temperature include pharmaceutically-acceptable glycerolipids. Glycerolipids are composed mainly of mono-, di-, and trisubstituted glycerols. One group of glycerolipids is the glycerides, where one, two, or all three hydroxyl groups of glycerol are each esterified using a fatty acid to produce monoglycerides, diglycerides and triglycerides, respectively. In these compounds, each hydroxyl groups of glycerol may be esterified by the same fatty acid or different fatty acids. Additionally, glycerides may be acetylated to produce acetylated monoglycerides, acetylated diglycerides and acetylated triglycerides. The monoglyceride may include a saturated or unsaturated fatty acid having a carbon length of $C_{12}$-$C_{24}$. The diglyceride may include one saturated or unsaturated fatty acid having a carbon length of $C_{12}$-$C_{24}$, or two saturated or unsaturated fatty acids each having a carbon length of $C_{12}$-$C_{24}$. The triglyceride may include one saturated or unsaturated fatty acid having a carbon length of $C_{12}$-$C_{24}$, two saturated or unsaturated fatty acids each having a carbon length of $C_{12}$-$C_{24}$, or three saturated or unsaturated fatty acids each having a carbon length of $C_{12}$-$C_{24}$. Commercially available mixtures of pharmaceutically-acceptable glycerolipids include, without limitation, Cocoa butter, mixtures of PEG-6 sterate and ethylene glycol palmitostearate and PEG-32 stearate (TEFOSE® 1500; TEFOSE® 63), mixtures of triceteareth-4 phosphate and ethylene glycol palmitostearate and diethylene glycol palmitostearate (SEDEFOS® 75), mixtures of glycerol monostearate and PEG-75 stearate (GELOT®), mixtures of cetyl alcohol and ethoxylated fatty alcohols (seteth-2-, steareth-20) (EMULCIRE®), mixtures of saturated $C_{10}$-$C_{18}$ triglycerides having a melting point around 33°C (GELUCIRE® 33/01), mixtures of saturated $C_{10}$-$C_{18}$ triglycerides having a melting point around 39°C (GELUCIRE® 39/01), mixtures of saturated $C_{10}$-$C_{18}$ triglycerides having a melting point around e.g. 43°C (GELUCIRE® 43/01), mixtures of glycerol monostearate 40-55 (type I) and diglycerides (GELEOL® Mono and Diglycerides), and mixtures of medium-chain triglycerides (LABRAFAC® Lipophile WL 1349).

Other suitable room temperature solid lipids include pharmaceutically-acceptable glycol fatty acid esters, such a monoester of a glycol, a diester of a glycol, or a triester of a glycol. A glycol fatty acid ester may include, without limitation, an ethylene glycol fatty acid ester, a diethylene glycol fatty acid ester, a propylene glycol fatty acid ester, and a dipropylene fatty acid ester. Commercially available pharmaceutically-acceptable glycol fatty acid esters include, without limitation, propylene glycol monopalmitostearate (MONOSTEOL®), propylene glycol dicaprylocaprate (LABRAFAC® PG), propylene glycol monolaurate (type I) (LAUROGLYCOL® FCC), propylene glycol monolaurate (type II) (LAUROGLYCOL® 90), propylene glycol monocaprylate (type I) (CAPRYOL® PGMC), and propylene glycol monocaprylate (type II) (CAPRYOL® 90).

A further example of a suitable room temperature solid lipid may be a pharmaceutically-acceptable polyether fatty acid ester. A pharmaceutically-acceptable polyether fatty acid ester can be a mono-fatty acid ester of a polyether, a di-fatty acid ester of a polyether, or a tri-fatty acid ester of a polyether. Commercially available pharmaceutically-acceptable polyether fatty acid esters include, without limitation, caprylocaproyl macrogol-8 glycerides (LABRASOL®), PEG-8 beeswax (APIFIL®), lauroyl macrogol-32 glycerides (GELUCIRE® 44/14), stearoyl macrogol-32 glycerides (GELUCIRE® 50.13), linoleoyl macrogol-6 glycerides (LABRAFIL® M2125CS), oleoyl macrogol-6 glycerides (LABRAFIL® M1944CS), and lauroyl macrogol-6 glycerides (LABRAFIL® M2130CS).

In preferred embodiments of the invention, the lipid that is solid at room temperature is selected from a glycerolipid, a glycol fatty acid esters, a polyether fatty acid esters or a glyceride. More preferably, the lipid comprises a mixture of saturated $C_{10}$-$C_{18}$ triglycerides, especially such a mixture having a melting point around 43°C.

Very suitably, the amount of the lipid component that is liquid at room temperature is from about 23% to about 27% by weight of the composition, more preferably about 24% to about 26% by weight of the composition, and about 25% by weight of the composition in a specific example described in more detail below.

Suitable lipids which are liquid at room temperature include a wide group of compounds that are generally soluble in organic solvents and generally insoluble in water. Typical mixtures of pharmaceutically-acceptable room temperature liquid lipids include a mixture of one or more fatty acids, a mixture of one or more partially hydrolyzed fats, and a mixture of one or more partially hydrogenated fats.

The process of hydrogenation adds hydrogen atoms to unsaturated lipid, eliminating double bonds and making them into partially or completely saturated lipid. Partial hydrogenation is a chemical rather than enzymatic, that converts a part of cis-isomers into trans-unsaturated lipids instead of hydrogenating them completely. In the first reaction step, one hydrogen is added, with the other, coordinatively unsaturated, carbon being attached to the catalyst. The second step is the addition of hydrogen to the remaining carbon, producing a saturated fatty acid. The first step is reversible, such that the hydrogen is re-adsorbed on the catalyst and the double bond is reformed. The intermediate with only one hydrogen added contains no double bond and can freely rotate. Thus, the double bond can re-form as either cis- or trans-, of which trans- is favoured, regardless the starting material.

Examples of a pharmaceutically-acceptable room temperature liquid lipids include monoglycerides including, without limitation, glycerol monomyristoleate, glycerol monopalmitoleate, glycerol monosapienate, glycerol monooleate, glycerol monoelaidate, glycerol monovaccenate, glycerol monolinoleate, glycerol monolinoelaidate, glycerol monolinolenate, glycerol monostearidonate, glycerol monoeicosenoate, glycerol monomeadate, glycerol monoarachidonate, glycerol monoeicosapentaenoate, glycerol monoerucate, glycerol monodocosahexaenoate, and glycerol mononervonate.

Commercially available pharmaceutically-acceptable room temperature liquid lipids include, without limitation, glyceryl dibehenate (COMPRITOL® 888), glycerol behenate (COMPRITOL® E ATO), glycerol dipalmitostearate (Biogapress Vegetal BM297ATO), glycerol distearate (type I) (PRECIROL® ATO 5), and glycerol monolinoleate (MAISINE™ 35-1).

In preferred embodiments, the lipid comprises a derivative of monolinoleate, especially glyceryl monolinoleate.

The composition also includes a stabilizing agent comprising a liquid glycol polymer. A stabilizing agent is a compound that interacts with a free acid or base present on a therapeutic compound, namely the NSAID, to shield the charges, thereby impeding ionic interactions between therapeutic compound/lipid matrices preventing the alignments necessary to form a crystalline matrix of a solid phase composition. Thus, a stabilizing agent prevents the thermodynamic transition of a composition into a classic solid phase or prolongs this transition to such an extent that it does not occur. The stabilizing agent is not a solvent as it is used in an amount that does not result in substantial dissolving of a solute.

The glycol polymer may comprise a pharmaceutically-acceptable PEG polymer. PEG polymers, also known as polyethylene oxide (PEO) polymers or polyoxyethylene (POE) polymers, are prepared by polymerization of ethylene oxide and are commercially available over a wide range of molecular weights from 100 g/mol to 10,000,000 g/mol. PEG polymers with a low molecular mass are liquids or low-melting solids, whereas PEG polymers of a higher molecular mass are solids. PEG polymers include, without limitation, PEG 100, PEG 200, PEG 300, PEG 400, PEG 500, PEG 600, PEG 700, PEG 800, PEG 900, PEG 1000, PEG 1100, PEG 1200, PEG 1300, PEG 1400, PEG 1500, PEG 1600, PEG 1700, PEG 1800, PEG 1900, PEG 2000, PEG 2100, PEG 2200, PEG 2300, PEG 2400, PEG 2500, PEG 2600, PEG 2700, PEG 2800, PEG 2900, PEG 3000, PEG 3250, PEG 3350, PEG 3500, PEG 3750, PEG 4000, PEG 4250, PEG 4500, PEG 4750, PEG 5000, PEG 5500, PEG 6000, PEG 6500, PEG 7000, PEG 7500, PEG 8000, PEG 8500, PEG 9000, PEG 9500, PEG 10,000, PEG 11,000, PEG 12,000, PEG 13,000, PEG 14,000, PEG 15,000, PEG 16,000, PEG 17,000, PEG 18,000, PEG 19,000, or PEG 20,000.

Alternatively the glycol polymer may comprise a pharmaceutically-acceptable polypropylene glycol (PPG) polymer. PPG polymers, also known as polypropylene oxide (PPO) polymers or polyoxypropylene (POP) polymers, are prepared by polymerization of propylene oxide and are commercially available over a wide range of molecular weights from 100 g/mol to 10,000,000 g/mol. PPG polymers with a low molecular mass are liquids or low-melting solids, whereas PPG polymers of a higher molecular mass are solids. PPG polymers include, without limitation, PPG 100, PPG 200, PPG 300, PPG 400, PPG 500, PPG 600, PPG 700, PPG 800, PPG 900, PPG 1000, PPG 1100, PPG 1200, PPG 1300, PPG 1400, PPG 1500, PPG 1600, PPG 1700, PPG 1800, PPG 1900, PPG 2000, PPG 2100, PPG 2200, PPG 2300, PPG 2400, PPG 2500, PPG 2600, PPG 2700, PPG 2800, PPG 2900, PPG 3000, PPG 3250, PPG 3350, PPG 3500, PPG 3750, PPG 4000, PPG 4250, PPG 4500, PPG 4750, PPG 5000, PPG 5500, PPG 6000, PPG 6500, PPG 7000, PPG 7500, PPG 8000, PPG 8500, PPG 9000, PPG 9500, PPG 10,000, PPG 11,000, PPG 12,000, PPG 13,000, PPG 14,000, PPG 15,000, PPG 16,000, PPG 17,000, PPG 18,000, PPG 19,000, or PPG 20,000.

Vey suitably, the amount of stabilizing agent is from about 8% to about 12% by weight of the composition, preferably about 9% to about 11% by weight of the composition, and about 10% by weight of the composition in a specific example described in more detail below.

In aspects of this embodiment, a solid solution composition comprises about 25% to about 31% by weight of therapeutic compound, about 34% to about 40% by weight of room temperature solid lipid or hard fat, about 22% to about 28% by weight of room temperature liquid lipid, and about 7% to about 13% of a stabilizing agent. In aspects of this embodiment, a solid solution composition comprises about 26% to about 30% by weight of therapeutic compound, about 35% to about 39% by weight of room temperature solid lipid or hard fat, about 23% to about 27% by weight of room temperature liquid lipid, and about 8% to about 12% of a liquid glycol polymer and/or a monohydric alcohol, and/or isosorbide dimethyl ether, and/or diethylene glycol monoethyl ether (2-(2-ethoxyethoxy)ethanol). In other aspects of this embodiment, a solid solution composition comprises about 27% to about 29% by weight of therapeutic compound, about 36% to about 38% by weight of room temperature solid lipid or hard fat, about 24% to about 26% by weight of room temperature liquid lipid, and about 9% to about 11 % of a liquid glycol polymer and/or a monohydric alcohol, and/or isosorbide dimethyl ether, and/or diethylene glycol monoethyl ether (2-(2-ethoxyethoxy)ethanol). In aspects of this embodiment, a solid solution composition comprises about 25% to about 31% by weight of a NSAID, about 34% to about 40% by weight of room temperature solid lipid or hard fat that is a triglyceride mixture, about 22% to about 28% by weight of room temperature liquid lipid that is a monoglyceride, and about 7% to about 13% of a liquid PEG polymer. In aspects of this embodiment, a solid solution composition comprises about 26% to about 30% by weight of a NSAID, about 35% to about 39% by weight of room temperature solid lipid or hard fat that is a triglyceride mixture, about 23% to about 27% by weight of room temperature liquid lipid that is a monoglyceride, and about 8% to about 12% of a liquid PEG polymer. In other aspects of this embodiment, a solid solution composition comprises about 27% to about 29% by weight of a NSAID, about 36% to about 38% by weight of room temperature solid lipid or hard fat that is a triglyceride mixture, about 24% to about 26% by weight of room temperature liquid lipid that is a monoglyceride, and about 9% to about 11% of a liquid PEG polymer. A room temperature solid lipid or hard fat that is a triglyceride mixture can be a GELUCIRE® 43/01. A room temperature liquid lipid that is a monoglyceride can be MAISINE™ 35-1. A liquid PEG polymer can be a PEG 100, a PEG 200, a PEG 300, a PEG 400, a PEG 500, a PEG 600, a PEG 700, a PEG 800, a PEG 900 or a PEG 1000.

In aspects of this embodiment, a solid solution composition comprises about 25% to about 31% by weight of a NSAID, about 34% to about 40% by weight of room temperature solid lipid or hard fat that is a triglyceride mixture, about 22% to about 28% by weight of room temperature liquid lipid that is a monoglyceride, and about 7% to about 13% of a liquid PEG polymer having a weight between 100 g/mol to 1000 g/mol. In aspects of this embodiment, a solid solution composition comprises about 26% to about 30% by weight of a NSAID, about 35% to about 39% by weight of room temperature solid lipid or hard fat that is a triglyceride mixture, about 23% to about 27% by weight of room temperature liquid lipid that is a monoglyceride, and about 8% to about 12% of a liquid PEG polymer having a weight between 100 g/mol to 1000 g/mol. In other aspects of this embodiment, a solid solution composition comprises about 27% to about 29% by weight of a NSAID, about 36% to about 38% by weight of room temperature solid lipid or hard fat that is a triglyceride mixture, about 24% to about 26% by weight of room temperature liquid lipid that is a monoglyceride, and about 9% to about 11% of a liquid PEG polymer having a weight between 100 g/mol to 1000 g/mol. A room temperature solid lipid or hard fat that is a triglyceride mixture can be a GELUCIRE®43/01. A room temperature liquid lipid that is a monoglyceride can be MAISINE™ 35-1. A liquid PEG polymer having a weight between 100 g/mol to 1000 g/mol can be a PEG 100, a PEG 200, a PEG 300, a PEG 400, a PEG 500, a PEG 600, a PEG 700, a PEG 800, a PEG 900 or a PEG 1000.

In aspects of this embodiment, a solid solution composition comprises about 25% to about 31 % by weight of a propionic derived NSAID, about 34% to about 40% by weight of room temperature solid lipid or hard fat having a melting point of between 41 °C to 45°C and comprising a mixture of saturated $C_{10}$-$C_{18}$ triglycerides, about 22% to about 28% by weight of room temperature liquid lipid that is a glyceryl monolinoleate, and about 7% to about 13% of a liquid PEG polymer. In aspects of this embodiment, a solid solution composition comprises about 26% to about 30% by weight of a propionic derived NSAID, about 35% to about 39% by weight of room temperature solid lipid or hard fat having a melting point of between 41°C to 45°C and comprising a mixture of saturated $C_{10}$-$C_{18}$ triglycerides, about 23% to about 27% by weight of room temperature liquid lipid that is a glyceryl monolinoleate, and about 8% to about 12% of a liquid PEG polymer. In other aspects of this embodiment, a solid solution composition comprises about 27% to about 29% by weight a propionic derived NSAID, about 36% to about 38% by weight of room temperature solid lipid or hard fat having a melting point of between 41 °C to 45°C and comprising a mixture of saturated $C_{10}$-$C_{18}$ triglycerides, about 24% to about 26% by weight of room temperature liquid lipid that is a glyceryl monolinoleate, and about 9% to about 11% of a liquid PEG polymer. A propionic acid derived NSAID can be a Dexibuprofen, a Dexketoprofen, a Fenoprofen, a Flurbiprofen, an Ibuprofen, a Ketoprofen, a Loxoprofen, a Naproxen or an Oxaprozin. A room temperature solid lipid or hard fat having a melting point of between 41°C to 45°C and comprising a mixture of saturated $C_{10}$-$C_{18}$ triglycerides can be a GELUCIRE® 43/01. A room temperature liquid lipid that is a glyceryl monolinoleate can be MAISINE™ 35-1. A liquid PEG polymer can be a PEG 100, a PEG 200, a PEG 300, a PEG 400, a PEG 500, a PEG 600, a PEG 700, a PEG 800, a PEG 900 or a PEG 1000.

In aspects of this embodiment, a solid solution composition comprises about 25% to about 31 % by weight of a propionic derived NSAID, about 34% to about 40% by weight of room temperature solid lipid or hard fat having a melting point of between 41°C to 45°C and comprising a mixture of saturated $C_{10}$-$C_{18}$ triglycerides, about 22% to about 28% by weight of room temperature liquid lipid that is a glyceryl monolinoleate, and about 7% to about 13% of a liquid PEG polymer having a weight between 100 g/mol to 1000 g/mol. In aspects of this embodiment, a solid solution composition comprises about 26% to about 30% by weight of a propionic derived NSAID, about 35% to about 39% by weight of room temperature solid lipid or hard fat having a melting point of between 41°C to 45°C and comprising a mixture of

saturated $C_{10}$-$C_{18}$ triglycerides, about 23% to about 27% by weight of room temperature liquid lipid that is a glyceryl monolinoleate, and about 8% to about 12% of a liquid PEG polymer having a weight between 100 g/mol to 1000 g/mol. In other aspects of this embodiment, a solid solution composition comprises about 27% to about 29% by weight a propionic derived NSAID, about 36% to about 38% by weight of room temperature solid lipid or hard fat having a melting point of between 41 °C to 45°C and comprising a mixture of saturated $C_{10}$-$C_{18}$ triglycerides, about 24% to about 26% by weight of room temperature liquid lipid that is a glyceryl monolinoleate, and about 9% to about 11% of a liquid PEG polymer having a weight between 100 g/mol to 1000 g/mol. A propionic acid derived NSAID can be a Dexibuprofen, a Dexketoprofen, a Fenoprofen, a Flurbiprofen, an Ibuprofen, a Ketoprofen, a Loxoprofen, a Naproxen or an Oxaprozin. A room temperature solid lipid or hard fat having a melting point of between 41 °C to 45°C and comprising a mixture of saturated $C_{10}$-$C_{18}$ triglycerides can be a GELUCIRE® 43/01. A room temperature liquid lipid that is a glyceryl monolinoleate can be MAISINE™ 35-1. A liquid PEG polymer having a weight between 100 g/mol to 1000 g/mol can be a PEG 100, a PEG 200, a PEG 300, a PEG 400, a PEG 500, a PEG 600, a PEG 700, a PEG 800, a PEG 900 or a PEG 1000.

In aspects of this embodiment, a solid solution composition comprises about 25% to about 31 % by weight of a propionic derived NSAID, about 34% to about 40% by weight of room temperature solid lipid or hard fat having a melting point of between 42°C to 44°C and comprising a mixture of saturated $C_{10}$-$C_{18}$ triglycerides, about 22% to about 28% by weight of room temperature liquid lipid that is a glyceryl monolinoleate, and about 7% to about 13% of a liquid PEG polymer having a weight between 100 g/mol to 1000 g/mol. In aspects of this embodiment, a solid solution composition comprises about 26% to about 30% by weight of a propionic derived NSAID, about 35% to about 39% by weight of room temperature solid lipid or hard fat having a melting point of between 42°C to 44°C and comprising a mixture of saturated $C_{10}$-$C_{18}$ triglycerides, about 23% to about 27% by weight of room temperature liquid lipid that is a glyceryl monolinoleate, and about 8% to about 12% of a liquid PEG polymer having a weight between 100 g/mol to 1000 g/mol. In other aspects of this embodiment, a solid solution composition comprises about 27% to about 29% by weight a propionic derived NSAID, about 36% to about 38% by weight of room temperature solid lipid or hard fat having a melting point of between 42°C to 44°C and comprising a mixture of saturated $C_{10}$-$C_{18}$ triglycerides, about 24% to about 26% by weight of room temperature liquid lipid that is a glyceryl monolinoleate, and about 9% to about 11% of a liquid PEG polymer having a weight between 100 g/mol to 1000 g/mol. A propionic acid derived NSAID can be a Dexibuprofen, a Dexketoprofen, a Fenoprofen, a Flurbiprofen, an Ibuprofen, a Ketoprofen, a Loxoprofen, a Naproxen or an Oxaprozin. A room temperature solid lipid or hard fat having a melting point of between 42°C to 44°C and comprising a mixture of saturated $C_{10}$-$C_{18}$ triglycerides can be a GELUCIRE® 43/01. A room temperature liquid lipid that is a glyceryl monolinoleate can be MAISINE™ 35-1. A liquid PEG polymer having a weight between 100 g/mol to 1000 g/mol can be a PEG 100, a PEG 200, a PEG 300, a PEG 400, a PEG 500, a PEG 600, a PEG 700, a PEG 800, a PEG 900 or a PEG 1000.

In aspects of this embodiment, a solid solution composition comprises about 25% to about 31% by weight of an Ibuprofen, about 34% to about 40% by weight of room temperature solid lipid or hard fat having a melting point of between 41°C to 45°C and comprising a mixture of saturated $C_{10}$-$C_{18}$ triglycerides, about 22% to about 28% by weight of room temperature liquid lipid that is a glyceryl monolinoleate, and about 7% to about 13% of a liquid PEG polymer. In aspects of this embodiment, a solid solution composition comprises about 26% to about 30% by weight of an Ibuprofen, about 35% to about 39% by weight of room temperature solid lipid or hard fat having a melting point of between 41°C to 45°C and comprising a mixture of saturated $C_{10}$-$C_{18}$ triglycerides, about 23% to about 27% by weight of room temperature liquid lipid that is a glyceryl monolinoleate, and about 8% to about 12% of a liquid PEG polymer. In other aspects of this embodiment, a solid solution composition comprises about 27% to about 29% by weight an Ibuprofen, about 36% to about 38% by weight of room temperature solid lipid or hard fat having a melting point of between 41 °C to 45°C and comprising a mixture of saturated $C_{10}$-$C_{18}$ triglycerides, about 24% to about 26% by weight of room temperature liquid lipid that is a glyceryl monolinoleate, and about 9% to about 11% of a liquid PEG polymer. A room temperature solid lipid or hard fat having a melting point of between 41°C to 45°C can be a GELUCIRE®43/01. A room temperature liquid lipid that is a glyceryl monolinoleate can be MAISINE™ 35-1. A liquid PEG polymer can be a PEG 100, a PEG 200, a PEG 300, a PEG 400, a PEG 500, a PEG 600, a PEG 700, a PEG 800, a PEG 900 or a PEG 1000.

In aspects of this embodiment, a solid solution composition comprises about 25% to about 31% by weight of an Ibuprofen, about 34% to about 40% by weight of room temperature solid lipid or hard fat having a melting point of between 42°C to 44°C and comprising a mixture of saturated $C_{10}$-$C_{18}$ triglycerides, about 22% to about 28% by weight of room temperature liquid lipid that is a glyceryl monolinoleate, and about 7% to about 13% of a liquid PEG polymer. In aspects of this embodiment, a solid solution composition comprises about 26% to about 30% by weight of an Ibuprofen, about 35% to about 39% by weight of room temperature solid lipid or hard fat having a melting point of between 42°C to 44°C and comprising a mixture of saturated $C_{10}$-$C_{18}$ triglycerides, about 23% to about 27% by weight of room temperature liquid lipid that is a glyceryl monolinoleate, and about 8% to about 12% of a liquid PEG polymer. In other aspects of this embodiment, a solid solution composition comprises about 27% to about 29% by weight an Ibuprofen, about 36% to about 38% by weight of room temperature solid lipid or hard fat having a melting point of between 42°C to 44°C and comprising a mixture of saturated $C_{10}$-$C_{18}$ triglycerides, about 24% to about 26% by weight of room temperature liquid lipid that is a glyceryl monolinoleate, and about 9% to about 11% of a liquid PEG polymer. A room temperature solid lipid or hard fat having a melting point of between 42°C to 44°C can be a GELUCIRE®43/01. A room temperature liquid lipid that is a glyceryl monolinoleate can be MAISINE™ 35-1. A liquid PEG polymer can be a PEG 100, a PEG 200, a PEG 300, a PEG 400, a PEG 500, a PEG 600, a PEG 700, a PEG 800, a PEG 900 or a PEG 1000.

In aspects of this embodiment, a solid solution composition comprises about 25% to about 31% by weight of an Ibuprofen, about 34% to about 40% by weight of room temperature solid lipid or hard fat having a melting point of between 41 °C to 45°C and comprising a mixture of saturated $C_{10}$-$C_{18}$ triglycerides, about 22% to about 28% by weight of room temperature liquid lipid that is a glyceryl monolinoleate, and about 7% to about 13% of a liquid PEG polymer having a weight between 100 g/mol to 1000 g/mol. In aspects of this embodiment, a solid solution composition comprises about 26% to about 30% by weight of an Ibuprofen, about 35% to about 39% by weight of room temperature solid lipid or hard fat having a melting point of between 41°C to 45°C and comprising a mixture of saturated $C_{10}$-$C_{18}$ triglycerides, about 23% to about 27% by weight of room temperature liquid lipid that is a glyceryl monolinoleate, and about 8% to about 12% of a liquid PEG polymer having a weight between 100 g/mol to 1000 g/mol. In other aspects of this embodiment, a solid solution composition comprises about 27% to about 29% by weight an Ibuprofen, about 36% to about 38% by weight of room temperature solid lipid or hard fat having a melting point of between 41 °C to 45°C and comprising a mixture of saturated $C_{10}$-$C_{18}$ triglycerides, about 24% to about 26% by weight of room temperature liquid lipid that is a glyceryl monolinoleate, and about 9% to about 11% of a liquid PEG polymer having a weight between 100 g/mol to 1000 g/mol. A room temperature solid lipid or hard fat having a melting point of between 41 °C to 45°C and comprising a mixture of saturated $C_{10}$-$C_{18}$ triglycerides can be a GELUCIRE® 43/01. A room temperature liquid lipid that is a glyceryl monolinoleate can be MAISINE™ 35-1. A liquid PEG polymer having a weight between 100 g/mol to 1000 g/mol can be a PEG 100, a PEG 200, a PEG 300, a PEG 400, a PEG 500, a PEG 600, a PEG 700, a PEG 800, a PEG 900 or a PEG 1000.

In aspects of this embodiment, a solid solution composition comprises about 25% to about 31% by weight of an Ibuprofen, about 34% to about 40% by weight of room temperature solid lipid or hard fat having a melting point of between 42°C to 44°C and comprising a mixture of saturated $C_{10}$-$C_{18}$ triglycerides, about 22% to about 28% by weight of room temperature liquid lipid that is a glyceryl monolinoleate, and about 7% to about 13% of a liquid PEG polymer having a weight between 100 g/mol to 1000 g/mol. In aspects of this embodiment, a solid solution composition comprises about 26% to about 30% by weight of an Ibuprofen, about 35% to about 39% by weight of room temperature solid lipid or hard fat having a melting point of between 42°C to 44°C and comprising a mixture of saturated $C_{10}$-$C_{18}$ triglycerides, about 23% to about 27% by weight of room temperature liquid lipid that is a glyceryl monolinoleate, and about 8% to about 12% of a liquid PEG polymer having a weight between 100 g/mol to 1000 g/mol. In other aspects of this embodiment, a solid solution composition comprises about 27% to about 29% by weight an Ibuprofen, about 36% to about 38% by weight of room temperature solid lipid or hard fat having a melting point of between 42°C to 44°C and comprising a mixture of saturated $C_{10}$-$C_{18}$ triglycerides, about 24% to about 26% by weight of room temperature liquid lipid that is a glyceryl monolinoleate, and about 9% to about 11% of a liquid PEG polymer having a weight between 100 g/mol to 1000 g/mol. A room temperature solid lipid or hard fat having a melting point of between 42°C to 44°C and comprising a mixture of saturated $C_{10}$-$C_{18}$ triglycerides can be a GELUCIRE® 43/01. A room temperature liquid lipid that is a glyceryl monolinoleate can be MAISINE™ 35-1. A liquid PEG polymer having a weight between 100 g/mol to 1000 g/mol can be a PEG 100, a PEG 200, a PEG 300, a PEG 400, a PEG 500, a PEG 600, a PEG 700, a PEG 800, a PEG 900 or a PEG 1000.

Specific embodiments of the invention provide a solid solution pharmaceutical composition comprising

a) 25% to 31% by weight ibuprofen;
b) 34% to 40% by weight of mixture of saturated $C_{10}$-$C_{18}$ triglycerides, which mixture is solid at room temperature, namely 20°C;
c) 22% to 28% by weight of a monoglyceride which is a liquid at room temperature, namely 20°C;
d) 7% to 13% by weight of a stabilizing agent comprising a polyoxyethylene polymer; and
e) 0.5% by weight or less of a hydrophilic solvent

wherein the composition is liquid at normal human body temperature, namely 37°C, and is a solid at room temperature, namely 20°C.

For administration of the compositions of the invention various dosage forms may be used. In preferred embodiments, illustrated in the example below, a capsule is provided containing the composition - note the calculation of % by weight above is with reference to the composition not including the capsule. Typically, the capsule is of gelatin and the final dosage form comprises sorbitol, mannitol and/or sorbitans and other components such as water and colouring.

In typical use, the composition is taken orally resulting in rapid and effective therapy.

The invention is now illustrated in specific embodiments with reference to the accompanying drawings in which:

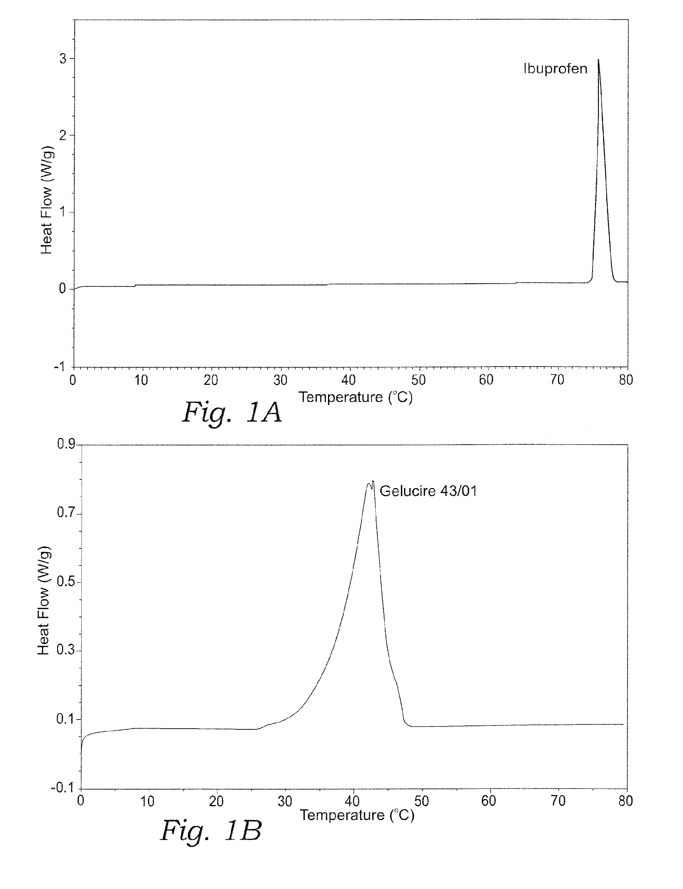

Fig. 1A shows a differential scanning calorimetry (DSC) graph of ibuprofen alone exhibiting a melting point range of 75°C to 78°C;

FIG. 1B shows a DSC graph of GELUCIRE® 43/01 alone exhibiting a melting point range of 41 °C to 45°C; and

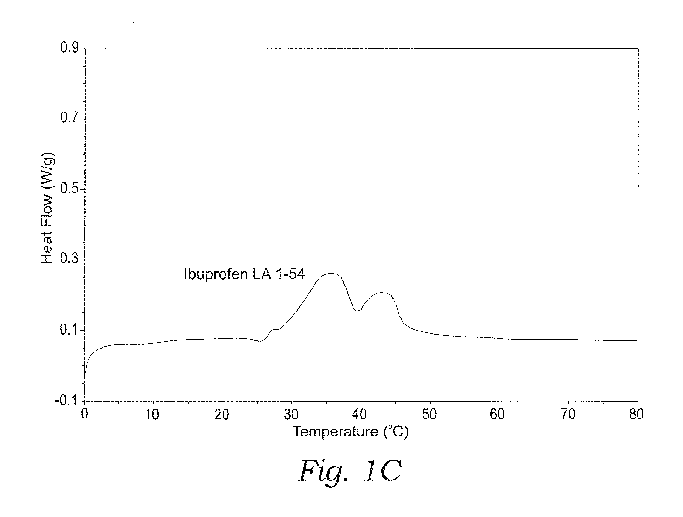

FIG. 1C shows a DSC graph of solid solution composition LA 1-54 comprising ibuprofen exhibiting a melting point range of 33°C to 37°C.

### Example 1 - Formulation

A quantity of 200g of Ibuprofen 25 EP was dissolved in 175.08g of a room temperature liquid lipid, namely glycerol monolinoleate EP, NF (commercially available as MAISINE™ 35-1). 72.3g of a stabilizer, Macrogol 400 EP (PEG 400 NF), was added and the mixture was heated to a temperature of between 50°C and 60°C.

262.62g of GELUCIRE® 43/1 (a room temperature solid lipid having a melting point of between 41°C and 45°C, and comprising a mixture of saturated $C_{10}$-$C_{18}$ triglycerides) was added and stirring was continued until the solid lipid was incorporated.

The mixture was divided into 1000 doses and allowed to cool and solidify.

After solidification the doses were coated in a gelatine based material, also incorporating sorbitol, mannitol, sorbitans and water. The coating was coloured and printed.

Each capsule therefore comprised:-

| Material | Amount/cap. (mg) | % by weight of total (including capsule components) |
|---|---|---|
| Fill | | |
| GELUCIRE® 43/01 | 262.62 | 25.52 |
| Ibuprofen 25 EP, USP | 200 | 19.44 |
| MAISINE™ 35-1 | 175 | 17.01 |
| Macrogol 400 EP | 72.3 | 7.03 |
| Total Fill | 710 | |
| | | |
| Shell | | |
| Gelatin EP, NF | 197.41 | 19.18 |
| Sorbitol, Mannitol, Sorbitans | 95.88 | 9.32 |
| Water | 25.02 | 2.43 |
| Opacode WB White NS-78-18011 (white printing ink) | 0.5 | 0.05 |
| Dualdustmaster FD&C blue #1 (Brilliant Blue FCF, E133) | 0.09 | 0.01 |
| Dualgran FD&C RED #40 (Allura Red, E129) | 0.09 | 0.01 |
| Total Shell | 319 | |
| | | |
| TOTAL | 1029 | 100 |

### Example 2 - Macrophage Uptake of Formulation

Cultures of U937 monocyte cell line were grown in RPMI-1640 supplemented with 10% fetal calf serum (FCS) until the cells reached 90% confluent monolayer. These cells were then treated with PMA and incubated in a 37°C incubator under 5% carbon dioxide until the cells differentiated into macrophages.

Monolayers of macrophages were washed with fresh medium and then 3 mL of one of the following test solutions was added:

1. A solid solution formulation of Example 1 (i.e. the Fill component) comprising ibuprofen, GELUCIRE® 43/01 (Gattefosse), MAISINE™ 35-1 (Gattefosse) and PEG 400;

2. Ibuprofen free acid; and
3. Vehicle with no therapeutic compound.

After incubation for 45 minutes the test solution supernatants were removed and saved for analysis, and the cells were washed in PBS several times and lysed using two cycles of freeze-thawing. Therapeutic compound concentration present in the test solution, test solution supernatant, and cell lysate fractions was measured by HPLC.
The percentage therapeutic compound taken up by the macrophages was calculated using the following formula:

$$\% \text{ therapeutic compound adsorbed} = 100 \times (\text{compound mass recovered from cell lysate}) / (\text{compound mass delivered in test solution} - \text{compound mass recovered from test solution supernatant}).$$

Results are shown in the Table 1 below. These results indicate that mean uptake of therapeutic compound by macrophages increases 600% using the formulation of Example 1, relative to ibuprofen free acid alone.

| Table 1. Macrophage Uptake of Therapeutic Compound | | |
|---|---|---|
| Formulation | Mean Mass Compound Uptake | % Increase Compound Uptake |
| 1 | 2.4% | 600% |
| 2 | 0.4% | - |
| 3 | 0.0% | - |

## Example 3 - DSC Analysis

Differential Scanning Calorimetry (DSC) was used to demonstrate the melting points of the various constituents in the claimed composition, the results being shown in Figure 1.
In Figure 1A, the DSC spectrum shows a reference peak for Ibuprofen alone. This demonstrates the characteristic melting point of Ibuprofen to be between 75 and 80 degrees Celsius. In Figure 1B, the DSC spectrum shows a reference peak for GELUCIRE® 43/01 alone. This demonstrates the characteristic melting point of GELUCIRE® 43/01 to be between 41 and 45 degrees Celsius. MAISINE™ 35-1 and PEG 400 are liquids at room temperature, and as such have melting points below 20°C. For example, MAISINE™ 35-1 has a melting point temperature range of about 14°C to about 16°C and PEG 400 has a melting point temperature range of about 4°C to about 8°C.
Figure 1C shows a DSC spectrum for the claimed composition. Surprisingly, despite Ibuprofen being present in the composition, there is no peak visible between 75 and 80 degrees Celsius. In addition, the GELUCIRE® 43/01 peak is significantly reduced. Instead the constituent peaks are observed between 33 and 37 degrees Celsius, demonstrating that the composition considerably lowers the melting point of Ibuprofen. Interestingly the newly observed melting point for Ibuprofen is close to human body temperature, i.e. 37 degrees Celsius.

## Example 4 - Clinical Trial Protocol

A clinical trial was developed as described below to test the safety and efficacy of the claimed composition in humans.
Healthcare professionals identify and recruit subjects for the trial according to specific inclusion criteria, e.g. male or female, 18-70 years of age, noticeable knee pain lasting over 48 hours at least twice during the previous year and a willingness to abstain from other analgesics including NSAIDs. Additionally, certain exclusion criteria are identified, e.g. pregnancy, history of serious illness, a BMI <18 or >39 or any analgesic other than paracetamol taken prior to study medication.
The relevant ethical approval is obtained for the study and subjects are required to sign an "Informed Consent Document".
An assessment of baseline pain is carried out within 24 hours of the subject informing the study centre of onset of a knee flare episode. The subject is provided with a patient diary and WOMAC questionnaire to record variations in knee pain following baseline assessment.
The subject is randomised in a double-blind manner and told to take two capsules, three times daily; one arm of the treatment being 2 x 200mg of the claimed composition, 3 times daily, another arm being 1 x 400mg of Ibuprofen with 1 x placebo, 3 times daily, and another arm being 2 x 400mg of Ibuprofen, 3 times daily.

After 5 days the subject returns to the study centre for an end of treatment assessment.

The end of treatment assessment may result in the end of the study for the subject, if pain from the knee flare episode has been resolved or under control sufficiently not to require additional treatment. If the subject is still in pain, and agrees to continue participating in the study, another 5 days of treatment, on the same arm, will commence and a new patient diary and questionnaire will be provided.

It is anticipated that a total of 438 subjects will be included in the trial (146 in each group). Due to an expected drop-out rate of 10%, the target number of recruited subjects will need to be 486 (162 in each group).

Data from the trial will show effectiveness of the formulations of the invention.

Accordingly, the present invention provides an improved pharmaceutical composition incorporating a NSAID.

**Claims**

1. A pharmaceutical composition comprising:

   a) 25% to 31% by weight of a non-steroidal anti-inflammatory drug (NSAID);
   b) 34% to 40% by weight of a lipid which is solid at room temperature, namely 20°C;
   c) 22% to 28% by weight of a lipid which is a liquid at room temperature, namely 20°C; and
   d) 7% to 13% by weight of a stabilizing agent comprising liquid glycol polymer.

2. A pharmaceutical composition as claimed in claim 1, wherein the NSAID is ibuprofen.

3. A pharmaceutical composition as claimed in claim 1 or claim 2, which is liquid at normal human body temperature, namely 37°C, and is a solid at room temperature, namely 20°C.

4. A pharmaceutical composition as claimed in any preceding claim, which comprises 0.5% by weight or less of a hydrophilic solvent.

5. A pharmaceutical composition as claimed in any preceding claim, comprising 27% to 29% by weight of (a)

6. A pharmaceutical composition as claimed in any preceding claim, comprising 36% to 38% by weight of (b)

7. A pharmaceutical composition as claimed in any preceding claim, comprising 23% to 25% by weight of (c)

8. A pharmaceutical composition as claimed in any preceding claim, comprising 9% to 11% by weight of (d).

9. A pharmaceutical composition as claimed in any preceding claim, wherein (b) comprises a glycerolipid, a glycol fatty acid esters, a polyether fatty acid esters or a glyceride.

10. A pharmaceutical composition as claimed in any preceding claim, wherein (c) comprises one or more fatty acids, one or more partially hydrolysed fats, or one or more partially hydrogenated fats.

11. A pharmaceutical composition as claimed in any preceding claim, wherein (d) comprises a liquid polyethylene glycol polymer of a liquid polypropylene glycol polymer.

12. A solid solution pharmaceutical composition as claimed in claim 11, wherein the polyethylene glycol polymer comprises PEG 100, PEG 200, PEG 300, PEG 400, PEG 500, PEG 600, PEG 700, PEG 800, PEG 900, PEG 1000, or any combination thereof.

13. A solid solution pharmaceutical composition according to any preceding claim, comprising

   a) 25% to 31 % by weight ibuprofen;
   b) 34% to 40% by weight of mixture of saturated $C_{10}$-$C_{18}$ triglycerides, which mixture is solid at room temperature, namely 20°C;
   c) 22% to 28% by weight of a monoglyceride which is a liquid at room temperature, namely 20°C;
   d) 7% to 13% by weight of a stabilizing agent comprising a polyoxyethylene polymer; and
   e) 0.5% by weight or less of a hydrophilic solvent

wherein the composition is liquid at normal human body temperature, namely 37°C, and is a solid at room temperature, namely 20°C.

**14.** An oral dosage form of the pharmaceutical composition of any one of claims 1-13.

**15.** The pharmaceutical composition of any one of claims 1-13 or the dosage form of claim 14, for use in treating inflammation, pain, or chronic inflammation.

*Fig. 1A*

*Fig. 1B*

Fig. 1C

Europäisches
Patentamt

European
Patent Office

Office européen
des brevets

## EUROPEAN SEARCH REPORT

Application Number

EP 15 18 0256

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/162988 A1 (BANNISTER ROBIN M [GB] ET AL) 12 June 2014 (2014-06-12) * the whole document * * paragraph [0356]; figures; examples 1,8,17 * ----- | 1-15 | INV. A61K47/10 A61K47/14 A61K9/14 A61K9/48 A61K31/192 |
| | | | TECHNICAL FIELDS SEARCHED (IPC) A61K |

The present search report has been drawn up for all claims

| Place of search | Date of completion of the search | Examiner |
|---|---|---|
| The Hague | 20 January 2016 | Palma, Vera |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**EP 3 127 556 A1**

## ANNEX TO THE EUROPEAN SEARCH REPORT
## ON EUROPEAN PATENT APPLICATION NO.

EP 15 18 0256

20-01-2016

| Patent document cited in search report | Publication date | Patent family member(s) | Publication date |
|---|---|---|---|
| US 2014162988 A1 | 12-06-2014 | NONE | |

For more details about this annex : see Official Journal of the European Patent Office, No. 12/82